# EUROPEAN PATENT SPECIFICATION

(11) **EP 2 981 232 B1**
(45) Date of publication and mention of the grant of the patent: **13.11.2024**
(21) Application number: 15779831.5
(22) Date of filing: 08.04.2015
(51) Int. Cl.: A61F 5/08

(54) **NASAL DILATOR WITH ELASTIC MEMBRANE STRUCTURE**
NASALER DILATOR MIT ELASTISCHER MEMBRANSTRUKTUR
DILATATEUR NASAL AVEC STRUCTURE DE MEMBRANE ÉLASTIQUE

(30) Priority: 17.04.2014 US 201461980814 P; 02.04.2015 US 201514677897
(43) Date of publication of application: 10.02.2016
(73) Proprietor: Horizon IP Tech, LLC, Honolulu, HI 96813 (US)
(72) Inventor: Ierulli, Joseph V., Vancouver, WA 98661 (US); Sinda, Edmund A., Sarasota, FL 34240 (US)
(74) Representative: Stanley, David William
(86) International application number: PCT/IB2015/052565
(87) International publication number: WO 2015/159186

(56) References cited:
- EP-A1- 1 033 118
- US-A- 5 890 486
- US-A1- 2008 184 995
- US-A1- 2011 000 483
- US-A1- 2011 166 594
- US-A1- 2011 166 594

## Description

### RELATED APPLICATIONS

The present application claims priority benefit to U.S. Provisional Patent Application No. 61/980,813 filed 17 April 2014.

### FIELD OF THE INVENTION

The present invention relates generally to medical devices, and more particularly to apparatus for and methods of supporting and stabilizing or dilating external tissue in humans. As disclosed and taught in the preferred embodiments, the tissue dilator devices are particularly suitable for, and are directed primarily to, external nasal dilators for supporting, stabilizing, and dilating nasal outer wall tissues adjacent and overlying nasal airway passages of the human nose, including the nasal valve and the nasal vestibule areas thereof. The United States Food and Drug Administration classifies the external nasal dilator as a Class I Medical Device.

### BACKGROUND OF THE INVENTION

External nasal dilators worn on the skin surface of the human nose are well disclosed in the art. In use the external nasal dilator is flexed across the bridge of the nose, engaging the nasal passage outer wall tissues on each side of the bridge, and held thereto by adhesive.

A resilient member (synonymously referred to in the art as a *spring, spring member, resilient band, resilient member band, spring band,* or *bridge)* extends along the length of the device, secured to a thin, flexible sheet or sandwiched between two thin flexible sheets. Flexed across the bridge of the nose, the resilient member exerts spring biasing forces that urge the nasal outer wall tissues outward, stabilizing, expanding dilating the nasal passageways. Stabilized or dilated tissue reduces nasal airflow resistance within the nasal passages, promoting a corresponding increase, ease, or improvement in nasal breathing. Increased nasal airflow may have beneficial effects generally; for example, more restful or restorative sleep, or beneficial effects with regard to nasal congestion, nasal snoring or obstructive sleep apnea.

Further information pertaining to the prior art can be found in EP 1 033 118 that discloses, *inter alia,* a nasal dilator comprising a backing substrate having a first face and a second face, the first face provided with an adhesive layer and the second face provided with microcapsules containing volatile medication.

### SUMMARY OF THE INVENTION

Nasal dilator devices of the present invention comprise resilient and engagement elements. The engagement element functions primarily to affix, adhere, or engage the dilator to the skin surface of the nose overlying the nasal passages. The resilient element comprises a first part in the form of at least one resilient member that provides resiliency, or spring biasing forces, extending generally perpendicular to, or obliquely from, a surface plane thereof. The resilient element further comprises a second part in the form of an elastic membrane that provides elasticity, or stretching, tensing, or tensioning forces, extending generally parallel to a surface plane thereof. The dilator may also include a directional element that affects its spring biasing properties.

The elastic, or tensioning, force, directs dilator spring biasing, at least in part, as more fully described below. The dilator engagement element, by itself, provides little or no nasal dilation (although depending on the material used, could provide some stabilization to the outer walls of the nasal passages). The resilient element, by itself and affixed to the skin by adhesive, generally will not remain well engaged thereto. Accordingly, nasal dilators of the present invention preferably combine separate resilient and engagement elements in a single body truss.

Nasal dilators of the present invention are capable of resilient and elastic deformation by virtue of the resilient element. That is, when released after flexure the dilator returns to a substantially planar or pre-flexed state. In use the dilator stabilizes the nasal outer wall tissues and prevents the tissue from drawing inward during breathing. The dilator is further designed to expand, or dilate, the nasal passages. The dilator engages comfortably to the nose and is easily removed with little or no stress to the skin.

Dilators of the present invention generate a most preferred range of from about 15 grams to about 35 grams of resiliency, or spring biasing force, for non-athletes, and may generate from about 25 grams to about 45 grams for athletes. Less than 10 grams may not provide enough stabilization or dilation for some users, while greater than 35 grams may be uncomfortable for most home users. Overall spring biasing force is determined by the configuration of the resilient element: the width, length, and thickness of one or more resilient members, the type of resilient member material used, and the configuration of the elastic membrane and its material properties.

The dilator's resilient member(s) are semi-rigid; they flex out-of-plane and possess little or no in-plane stretch. The elastic membrane stretches in-plane, and may be flexible out of plane. The terms *spring biasing, spring biasing force, spring force, resiliency, spring constant,* etc. as used herein are generally synonymous, and apply primarily to the dilator's resilient member or members. The terms *stretch, tension, tensing, tensioning, elastic* or *elasticity* are intended herein to apply to the elastic membrane. Strictly speaking, the terms *resilient* or *resiliency* may be applied to an object that exhibits either 'flexure' or 'elasticity'. However, for purposes of the present invention 'resilient' applies herein exclusively to a resilient member or members. Specifically, a resilient member is resilient; it flexes out-of-plane. An elastic membrane is elastic; it stretches in-plane. Both return at least substantially to their initial position after *flexure* or *stretch,* respectively. Dilators are usually manufactured in the unflexed, unstretched, initial position, and are flexed and/or stretched during application. Thus, embodiments may be described either as "capable of" flexing and/or stretching (when the embodiment is in an initial or rest position) or "flexed" and/or "stretched" (when the embodiment is in use).

In the present invention, the elastic membrane directs the dilator's spring biasing properties, at least in part, to shift some delaminating peel and tensile forces generated by the resilient member(s) into shear forces extending somewhat parallel to the plane of the skin surface of the nose, both in a direction parallel to the dilator's length, and also perpendicular thereto, thus somewhat parallel to the bridge of the nose. The elastic membrane allows the resilient member(s) terminal ends to lift away from the surface of the nose, which creates less engagement contact surface area, and which may contribute to greater dilator comfort when in use. The elastic membrane and resilient member(s), combined, are the primary parts of the dilator's resilient element.

The resilient element comprises at least one resilient member extending at least through the intermediate region of the dilator, and may include two or more resilient members adjacent and generally parallel each other, or that may overlay entirely one onto another, or overlap partially one onto another. A single resilient member may include components, such as resilient spring fingers extending outward from a common center, or the resilient member may be bifurcated laterally to form resilient spring fingers extending away from the dilator's intermediate region to one or both end regions of the dilator.

### BRIEF DESCRIPTION OF THE SEVERAL VIEWS OF THE DRAWING

Fig. 1 is a plan view of a nasal dilator in accordance with the present invention.
Fig. 2 is a perspective view of the nasal dilator of Fig. 1, further illustrating a cover layer member.
Fig. 3 is an exploded perspective view of the nasal dilator of Fig. 2.
Fig. 4 is an end edge view of the dilator of Fig. 2, further illustrating a release liner backing.
Figs. 5 and 6 are cross sectional views, taken along the lines 5--5 in Fig. 2.
Fig. 7 is a perspective view of a portion of a face showing a front elevation, or long edge view, of the nasal dilator of Fig. 1 engaged to a human nose.
Fig. 8 is a plan view of a nasal dilator in accordance with the present invention.
Fig. 9 is a perspective view of the nasal dilator of Fig. 8.
Fig. 10 is an exploded perspective view of the nasal dilator of Fig. 9.
Fig. 11 is a three-quarter perspective view of a portion of a human nose showing the nasal dilator of Fig. 9 engaged thereon.
Fig. 12 is a plan view of a nasal dilator in accordance with the present invention.
Fig. 13 is a three-quarter perspective view of a portion of a human nose showing the nasal dilator of Fig. 12 engaged thereon.
Fig. 14 is a perspective view of the nasal dilator of Fig. 12.
Fig. 15 is an exploded perspective view of the nasal dilator of Fig. 13.
Fig. 16 is an exploded perspective view of an alternative form of the nasal dilator of Fig. 14.
Fig. 17 is a plan view of the contact and peripheral defining layers or members, respectively, of the nasal dilator of Fig. 1.
Fig. 18 is a plan view of the contact and peripheral defining layers or members, respectively, of the nasal dilator of Fig. 8.
Fig. 19 is a plan view of the contact and peripheral defining layers or members, respectively, of the nasal dilator of Fig. 12.
Fig. 20 is a plan view of a nasal dilator in accordance with the present invention.
Fig. 21 is a perspective view showing the nasal dilator of Fig. 20 engaged to a human nose.
Fig. 22 is a cross-sectional view taken along the lines 2--2 in Fig. 20 showing the dilator secured to a skin surface of a user, the view aligned with a fragmentary plan view of the end region corresponding thereto.
Fig. 23 is an exploded perspective view of the nasal dilator of Fig. 20.
Fig. 24 is a perspective view illustrating an alternative resilient element to that of Fig. 23A.
Fig. 25 is a plan view of a nasal dilator in accordance with the present invention.
Fig. 26 is a perspective view of the nasal dilator of Fig. 25.
Fig. 27 is an end edge view of the dilator of Fig. 25 secured to a skin surface of a user, the view aligned with a fragmentary plan view of the end region corresponding thereto.
Fig. 28 is an exploded perspective view of the nasal dilator of Fig. 25.
Fig. 29 is a plan view of a nasal dilator in accordance with the present invention.
Fig. 30 is a plan view showing an alternative resilient element to the nasal dilator of Fig. 29.
Fig. 31 is a perspective view showing the nasal dilator of Fig. 29 engaged to a human nose.
Fig. 32 is a fragmentary perspective view, taken on an enlarged scale, of a front elevation, or long edge view, of the nasal dilator of Fig. 29 engaged to the nose, the view aligned with a fragmentary plan view of the dilator end region corresponding thereto.
Fig. 33 is a perspective view of a nasal dilator in accordance with the present invention.
Fig. 34 is a three-quarter perspective view of a portion of a human nose showing the nasal dilator of Fig. 33 engaged thereon.
Fig. 35 is an exploded perspective view of the nasal dilator of Fig. 33.
Fig. 36 is a perspective view of a nasal dilator in accordance with the present invention.
Fig. 37 is a three-quarter perspective view of a portion of a human nose showing the nasal dilator of Fig. 36 engaged thereon.
Fig. 38 is an exploded perspective view of the nasal dilator of Fig. 36.

### DETAILED DESCRIPTION OF THE INVENTION

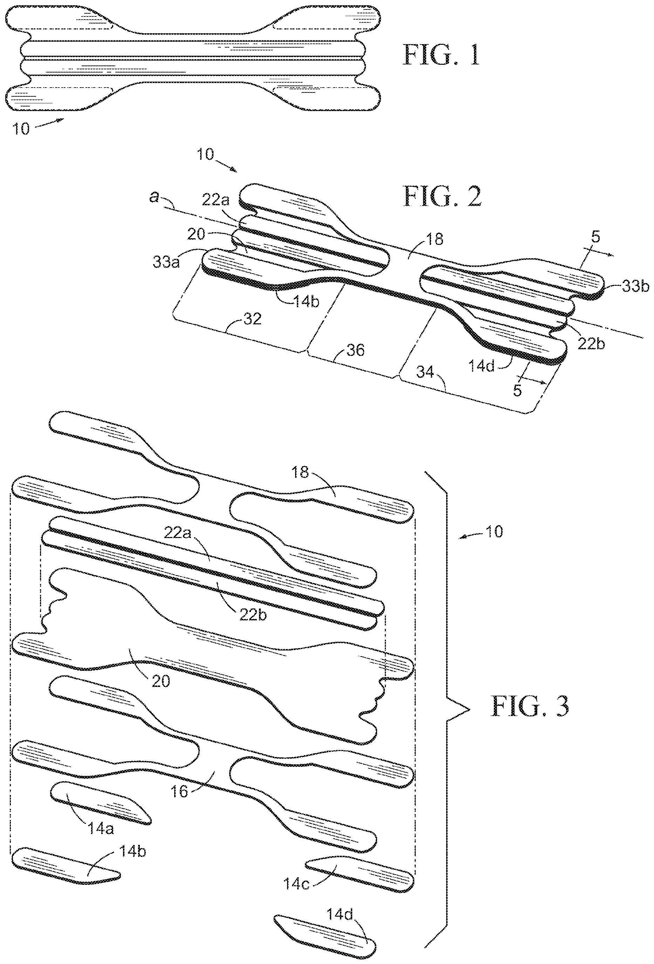

An embodiment of a form of a nasal dilator, **10,** in accordance with the present invention, is illustrated in Figs. 1-7. Dilator **10** comprises a laminate of several vertically stacked thin sheets, or layers, that correspond to the resilient and engagement elements. Dilator layers may include, for example, a contact layer, an elastic layer, and a resilient layer. The contact layer corresponds to the engagement element and provides the primary engagement element of the dilator. The elastic and resilient layers correspond to the resilient element. Dilator **10** may further include a cover layer, or a bonding layer. Dilator layers are preferably aligned along a longitudinal centerline, ***α*,** represented by a broken line as seen in Fig. 2.

A layer typically includes at least one member, and a member may further include one or more components. For example, the contact layer includes at least one contact member, **14,** and may alternatively include a plurality of discrete engagement contact points from a plurality of contact members (e.g., **14a** , **14b, 148c,** etc.). A layer may include several members, or parts, or a single member may be bisected or divided into two or more parts. As seen in Fig. 3, for example, the contact layer includes four discrete, spaced apart members, **14a-14d,** that provide four discrete engagement contact points for dilator **10** to engage the skin surface of the nose of a user.

The resilient element includes at least one resilient layer having at least one resilient member, **22** (if a plurality thereof, **22a** , **22b, 22c,** etc.). The elastic layer includes an elastic membrane, **20** (if a plurality thereof, **20a** , **20b, 20c,** etc.). An optional cover layer comprises at least one cover member, **18** (if a plurality thereof, **18a** , **18b, 18c,** etc.) For illustrative clarity Fig. 1 does not illustrate cover member **18,** and contact members **14** are represented by dashed lines.

The elastic layer may be substantially coextensive with the periphery of dilator **10.** It may define the dilator periphery, particularly in the absence of another layer doing so. Otherwise, the cover layer, bonding layer, or contact layer may define the dilator periphery, in whole or in part, or in combination with each other. Portions of any layer or member may overlap another layer or member.

A bonding layer member, **16** (if a plurality thereof, **16a, 16b,** etc.), may be used to secure any two layers or members together. For example, Fig. 3 shows bonding member **16** interposed between contact members **14** and elastic membrane **20.** Bonding member **16** may comprise an adhesive substance, such as, for example, a transfer or unsupported, adhesive. The bonding layer may also comprise a carrier material, or a carrier material with an adhesive substance disposed on one or both flat surface sides. Alternatively, the bonding member may comprise an adhesive disposed on a material layer such as that as may be used for the contact or cover members.

Dilator layers may be stacked, or vertically arranged, several different ways. Some layers, such as the contact, bonding, or cover layers, may be eliminated or interchanged.

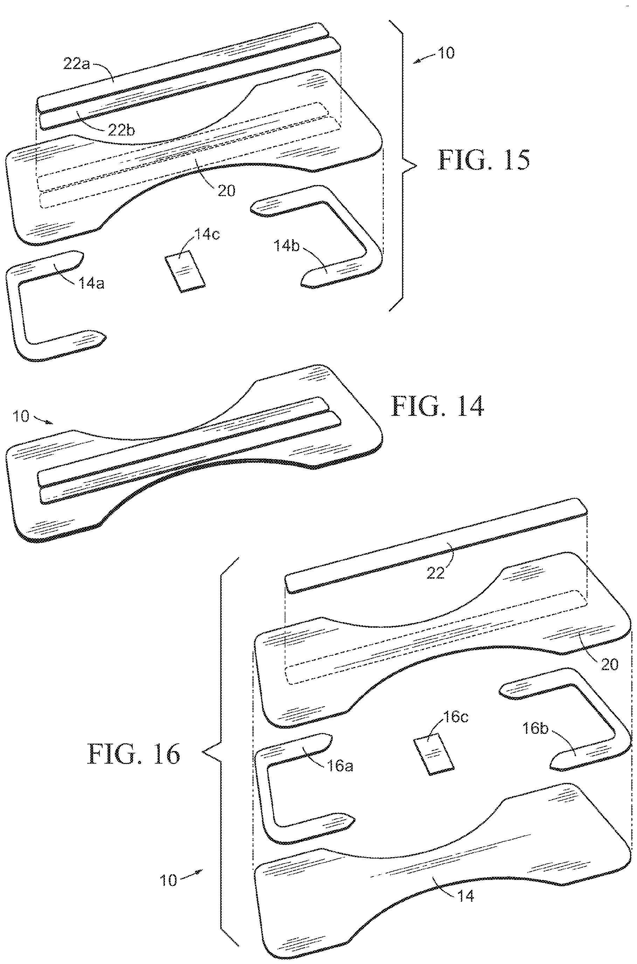

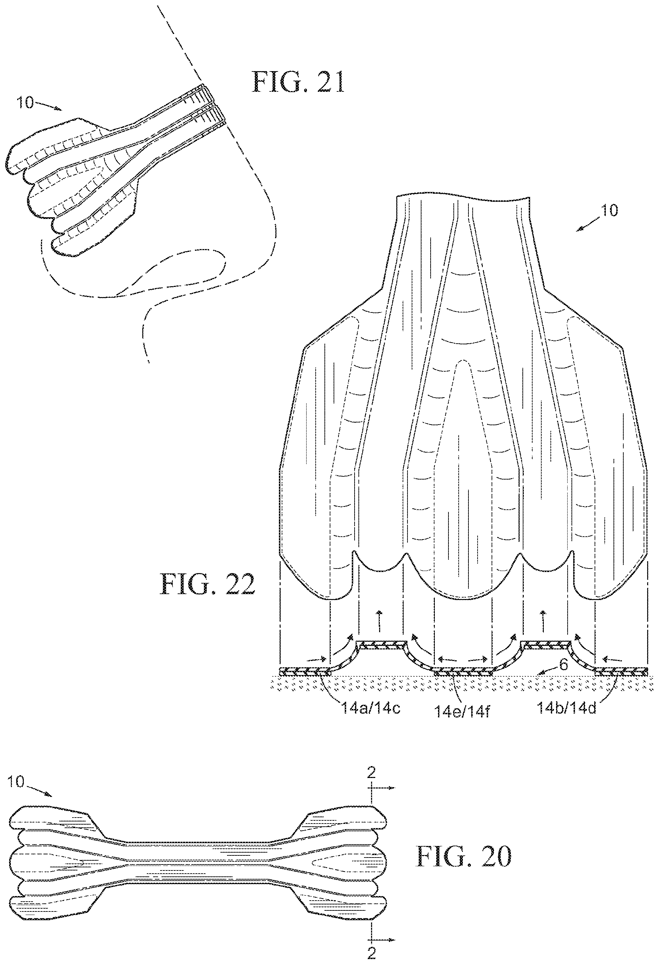

A portion of the elastic layer may contact the skin directly, in lieu of or in addition to a contact layer. The resilient layer may be positioned on top of or underneath the elastic layer. If the latter, both the contact member(s) and resilient member(s) would engage the same surface of the elastic membrane (as particularly depicted, for example, in Figs. 20-23). A cover layer may be used to sandwich the elastic layer between it and the contact layer. And similarly, a contact member **14** may be formed as a base layer (as seen, for example, in Fig. 16) that may define the periphery of dilator **10.**

Typically however, elastic membrane **20** is secured to contact member(s) **14** along the entire flat surface thereof opposite the skin-contact side. Similarly, elastic membrane **20** is most preferably secured to an entire flat surface side of resilient member(s) **22,** particularly where the resilient member is positioned on top of the elastic membrane. If included in dilator construction, cover member **18** is most preferably configured so that it does not extend across the resilient layer at each end region of the dilator, as seen in Fig. 2, for example, so that the end portions of the resilient element may flex and stretch away from the surface plane of dilator **10.**

Dilator layers or members may be secured to each other by any suitable means such as stitching or fastening, heat or pressure bonding or welding, ultrasonic welding, or the like, but are typically secured together by an adhesive substance disposed on at least a portion of one flat surface side of at least one member. In those instances where the resilient layer is on top-that is, uppermost-in the stacking order of dilator layers, it may be preferable to weld it to the layer to which it attaches or is primarily secured or affixed. A biocompatible adhesive for affixing or engaging dilator **10** to the skin of the nose is most preferably disposed on the skin engaging side of contact members **14** or other member or layer as may contact the skin.

The preferred materials for the contact and cover members may be selected from a range of widely available, preferably medical grade, flexible nonwoven synthetic fabrics or thermoplastic films that are preferably breathable and comfortable on the skin. Any suitable fabric or thermoplastic film, including various clear or colored films, including high Moisture Vapor Transmission Rate (MVTR) polyurethane film, may be used. A pressure sensitive adhesive, preferably biocompatible with external human tissue, may be disposed on at least one flat surface side of the material, in which case a protective, removable, release liner may cover the adhesive.

For use as the elastic membrane, a preferred material may be the same or similar to those for the contact and cover members, such as, for example, an ultra thin polyurethane film, a mesh, or a perforated material. The material preferably possesses in-plane elasticity, as discussed hereinbefore, particularly to a greater degree than, for example, that which may be present in materials chosen for the contact or cover members, and further having a range of elasticity that corresponds to the resiliency configured into the resilient member of dilator **10.** A pressure sensitive adhesive may be disposed on the skin-engaging side of the material, or on any material layer or portion thereof that will contact the skin.

The preferred material for the resilient member is a thermoplastic resin, which may be selected from a material class having a range of flexural, tensile and elastic moduli so as to have substantial in-plane rigidity and out-of-plane flexibility, such that the resilient member has suitable spring biasing properties at a thickness, for example, of from about 0.127 mm (0.005") to about 0.381 mm (0.015").

The most preferred thermoplastic material from said class is a biaxially oriented polyester resin, Poly(ethylene terephthalate), (PET or boPET). PET is used in a number of medical device applications, is particularly suitable for nasal dilator devices, is widely available as an industrial commodity, and comes in a variety of standard, off-the-shelf forms. However, any plastic film having the same or similar tensile, flexural, or elastic modulus values would also be suitable. The preferred material may include a pressure sensitive adhesive disposed on one or both surfaces thereof.

Contact member **14** may alternatively include or comprise a thermoplastic film selected from the preferred materials for resilient member **22,** which may be desirable, for example, where dilator **10** is configured to have a plurality of relatively small, discrete contact members **14.** The material is preferably thinner, however, and thus more flexible, than that used for the resilient member. The in-plane rigidity of the material may allow elastic membrane **20** to pull more equally or uniformly on the entirety of the flat surface of contact member **14,** and by extension, the skin surface engaged thereby. As a laminate, contact member **14** may comprise, for example, a spunlaced polyester fabric on the skin facing side together with a thin PET film on the opposite side.

A release liner may removably cover exposed adhesive from any layer preliminary to using the dilator. The shape and dimensions of the release liner may correspond to the periphery of dilator **10** or may exceed the periphery of one or more dilators **10.** The release liner may be bisected into two parts, which may overlap or abut, so as to facilitate removal from the dilator prior to use, as is common in adhesively applied medical devices.

As further seen in Fig. 2, the layers of dilator **10** form a unitary, or single body, truss. (Fig. 2 includes all of the layers illustrated in Fig. 3.) The truss has contiguous regions indicated generally by broken lines and brackets, including a first end region, **32,** a second end region, **34,** and an intermediate region, **36,** which joins first end region **32** to second end region **34.** The width of intermediate region **36** may be narrower than the width of end regions **32** and **34.** End regions **32** and **34** are adapted to engage outer wall tissues of the first and second nasal passages respectively. Each end region has a corresponding end edge, **33a** and **33b** extending between the respective long edges of dilator **10.** Portions of any layer may define a region of the truss or a portion thereof, and the layers, members or components of dilator **10** may extend from one region to another.

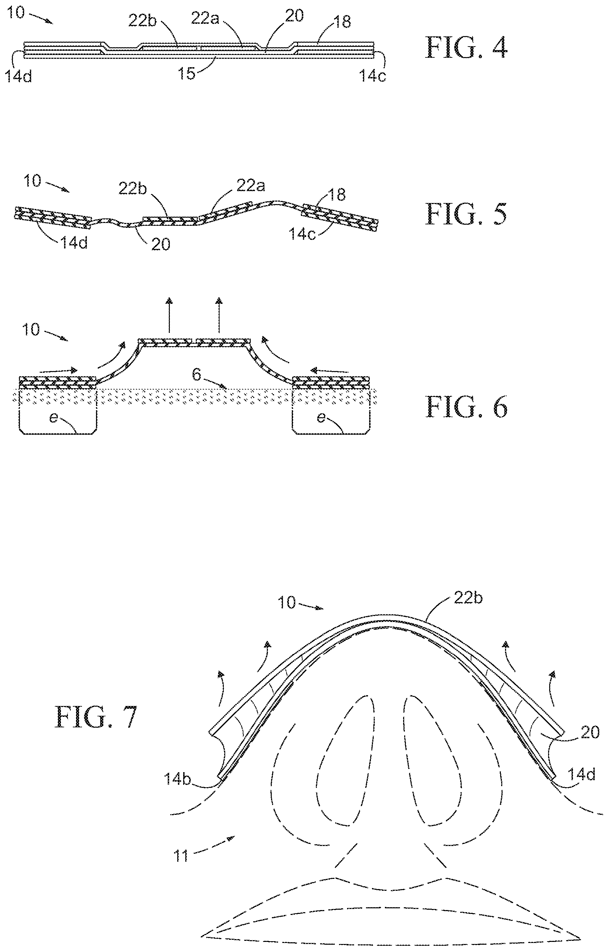

Figs. 4-6 illustrate dilator **10** in a supported, an unsupported, and an elastic or tensioned state, respectively. Fig. 4, which is an end edge view, shows dilator **10** removably secured to a release liner, **15,** which supports dilator **10** in a fixed, at-rest, position preliminary to use. The cross-sectional view of Fig. 5 illustrates that absent support from release liner **15,** or being in contact with the nose of a user, contact members **14** may move freely relative to resilient members **22,** the result of flexibility that may be inherent in the preferred material for elastic membrane **20.**

In use, however, as seen, for example, in Figs. 6 and 7, dilator **10** is in a tensioned and spring biasing state by virtue of being engaged to the nose of a user. Contact members **14** are affixed to the skin surface, as seen in the cross-sectional view of Fig. 6, at discrete engagement contact points, ***e*,** indicated by broken lines and brackets. Contact points ***e*** correspond to the surface area(s) of contact member(s) **14.**

As further seen in Figs. 6 and 7, when in use, dilator spring biasing forces exert a pulling force on contact members **14** as indicated by directional arrows. The elasticity inherent in elastic membrane **20** allows vertical separation of the resilient member terminal ends from skin surface, **6,** indicated by the two upwardly pointing arrows at the top center of Fig. 6. Directional arrows also indicate where said lifting creates at least some shear forces extending roughly parallel to skin surface **6.**

Spring biasing forces extend from contact points ***e*** along that portion of the surface plane of elastic membrane **20** extending between contact members **14** and resilient members **22.** Fig. 7 similarly illustrates portions of the resilient layer vertically separating from the skin surface, and the stretching of elastic membrane **20** when dilator **10** is engaged to a nose, **11.** It should be noted that Fig. 7, and similar drawing figures herein that show elastic membrane **20** under tension, include short curved lines to indicate said stretching for purposes of illustrative clarity, regardless whether the preferred material would or would not, in practice, exhibit the same or similar stretch lines.

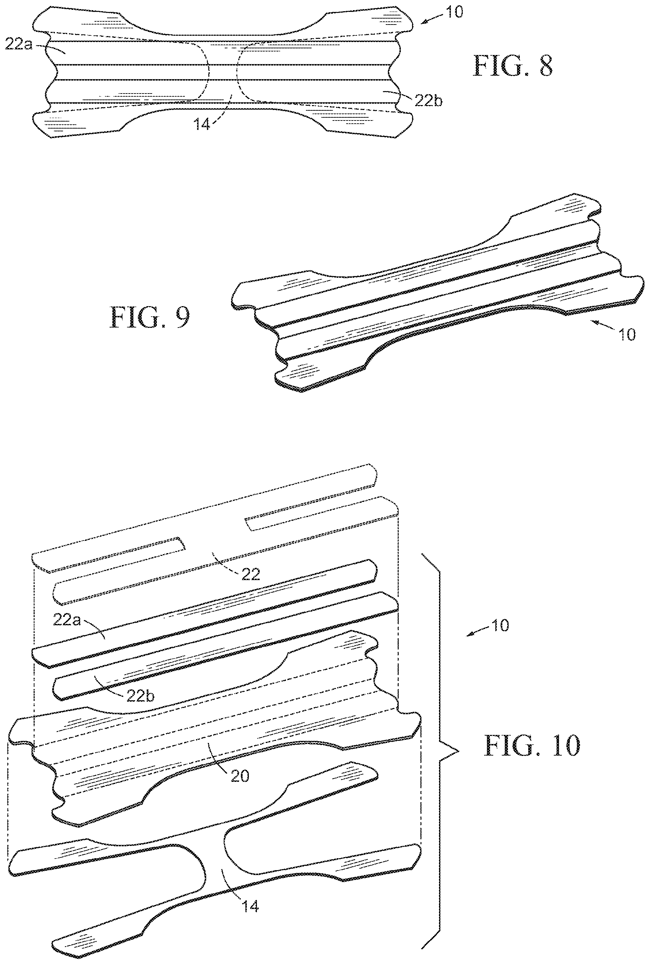

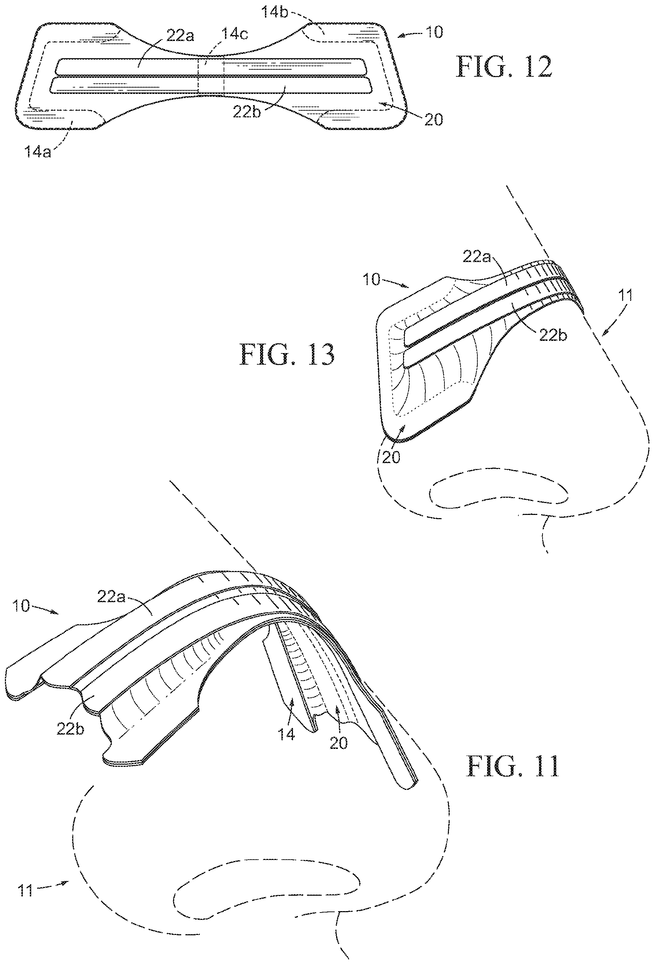

As seen in Figs. 8 and 10, a single contact member **14** (represented by dashed lines in Fig. 8) is configured to extend along the peripheral long edges of dilator **10,** and further to extend across the width of dilator **10** along at least a portion of intermediate region **36.** Fig. 11 illustrates dilator **10** affixed to nose **11.** (The nose is depicted in shadow lines as 'invisible', for illustrative clarity, such that a portion of the underside, or skin-engaging side, of dilator **10** may be seen.) The terminal ends of resilient members **22** separate from the skin at end regions **32** and **34.** Elastic membrane **20** is shown tensioned, exhibiting in-plane elasticity, or stretch, between contact member **14** and the respective outer long edges of resilient members **22a** and **22b.**

Figs. 8-11 illustrate resilient members **22a** and **22b** spaced further apart than, for example, the resilient members of dilator **10** shown in Figs. 1 and 12. That portion of elastic membrane **20** extending between the respective inside long edges of the resilient members, by virtue of its in-plane stretch, allows greater relative movement between the resilient members than if they were closer together, or otherwise secured to a material having less in-plane elasticity.

Fig. 10 further illustrates that the resilient layer of dilator **10** may alternatively comprise a single resilient member **22,** represented by dashed lines, configured to have a plurality of spring finger components extending outward from a common center. In the present embodiment the spring finger components are configured to emulate the effect of two parallel resilient members, but may otherwise be configured a variety of ways.

An embodiment of another form of dilator **10,** in accordance with the present invention, is illustrated in Figs. 12-15. The dilator end edges may be angled inward to correspond generally to the line where the nose meets the cheek of the user. Contact members **14a** and **14b** extend around the periphery of each end region **32** and **34,** respectively. The resilient members' terminal ends are set inboard from the dilator peripheral end edges such that elastic membrane **20** extends around and outboard thereof.

This configuration allows elastic membrane **20** to pull from the entire periphery of each end region, as particularly illustrated in Fig. 13, in which dilator **10** is depicted in use on nose **11.** Dashed lines indicate the margin between where elastic membrane **20a** is secured to contact member **14,** and that portion which separates from the surface of nose **11.** Contact member **14c** is positioned directly over the bridge of the nose, interposed between elastic membrane **20** and the skin surface thereat. Contact member **14c** may be non-adhering to the skin thereat, which may contribute to greater comfort.

As discussed hereinbefore, dilator layers may be configured and stacked several ways. Fig. 16 illustrates an alternative arrangement of dilator layers, for example, to that seen in Fig. 15. A base layer comprises a single contact member **14** having substantially the same peripheral shape as elastic membrane **20,** and thus substantially defining the periphery of dilator **10.** A plurality of bonding members, **16a-16c,** having substantially the same peripheral shape as contact members **14** as seen in Fig. 15, secure contact member **14** to elastic membrane **20.** And a single resilient member **22** is used in lieu of the two parallel resilient members as seen in Fig. 14.

Returning briefly now to separation of the resilient member terminal ends from the skin surface of the nose, as particularly seen in Figs. 6-7, 11 and 13 (and in other embodiments to follow): Said separation allows a change in the angle of focused spring biasing forces thereat, at least in part, shifting, or transforming, at least some of those forces from primarily peel and tensile forces into primarily shear forces.

Transformed forces are imparted to contact member(s) **14,** and by extension, to the end regions **32** and **34** of dilator **10.** This is in contrast to a greater delaminating tendency resulting from primarily peel forces. Shear forces are typically more easily withstood by the adhesives typically used to engage dilator **10** to the skin surface than are peel forces. Accordingly, a smaller amount of adhesive (or a less-aggressive adhesive) may suffice to secure dilator **10** to the nose, which may contribute to user comfort as well as lower the manufacturing cost of dilator **10.**

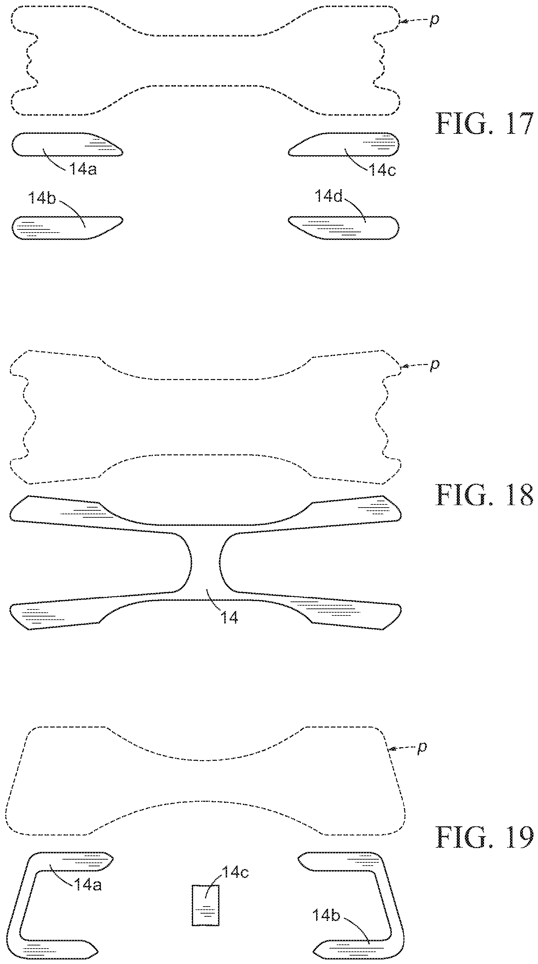

Resilient member terminal ends separating from the skin surface of the nose means less dilator surface area contacting the skin, particularly less surface area that is adhesively engaged to the skin. Less skin-contacting surface area may contribute to greater user comfort. Figs. 17-19 compares surface area between the overall periphery, ***p*,** of dilator **10** and the surface area of contact member(s) **14** actually touching the skin. Figs. 17-19 correspond to the dilators of Figs. 1, 8 and 12, respectively. Periphery ***p*** is depicted in broken lines, since the periphery of dilator **10** may be defined by a single layer or member, or a combination of dilator layers and/or members.

Using overall dimensions of roughly 69.85 mm (2.75") length, and from 19.05 mm to 24.13 mm (0.75" to 0.95") width, as may be typically found in the art, the total surface area of dilators 10, as defined by periphery p, as seen in Figs. 17-19, is roughly 9032.24,11612.9 and 8387.08 mm2 ( 1.4, 1.8 and 1.3 square inches), respectively. The surface area of contact member(s) **14** shown in Figs. 17-19 is roughly 296.77, 490.32 and 264.51 mm2 (0.46, 0.76 and 0.42 square inches), respectively, which is equal to about 32%, 43% and 32%, respectively, of the total surface areas of dilator **10.** Accordingly, contact member surface area could reasonably fall within a range of from about 25% to about 70% of the total surface area of dilator **10.**

Some embodiments of the present invention illustrate that resilient member(s) **22** may be affixed to the outer, or uppermost, side of elastic membrane **20.** However, one or more resilient members may be alternatively positioned underneath elastic membrane **20,** as seen, for example, in Figs. 20-23. Additionally (but not specifically shown in the drawing figures), elastic membrane **20** or bonding member **16** may be used to vertically separate or divide a plurality of resilient members into two resilient layers; for example, one or more affixed underneath and one or more affixed on top thereof.

Where positioned underneath elastic membrane **20,** resilient member(s) **22** may require little or no fixed engagement thereto. When under tension, as when secured to the nose, the resilient members would remain substantially held in place by the resilient membrane on top thereof. In such instances, the resilient member terminal ends may be removably secured to elastic membrane **20.** That is, secured in such a way as to be removed without compromising the structural integrity of the dilator. Such an arrangement may allow resilient members, of various dimensions or configuration, for example, to be interchanged by the user. Accordingly, partially assembled nasal dilators, absent resilient members, for example, may be provided in kit form for final assembly and custom configuration by the end user.

Similarly, various forms of the constituent members of dilator **10** of the present invention, including contact members, bonding members, elastic membranes, resilient and cover members, etc., may also be provided in kit form; the constituent parts adapted for assembly and configuration by the end user, including means for affixing the members to each other so as to assemble the constituent layers of dilator **10** at least substantially as described herein.

Figs. 1, 8 and 12 particularly illustrate the resilient layer of dilator **10** having two substantially parallel resilient members **22a** and **22b.** It will be apparent to those skilled in the art that a single resilient member **22** may be used in lieu thereof, as seen, for example, in Fig. 16.

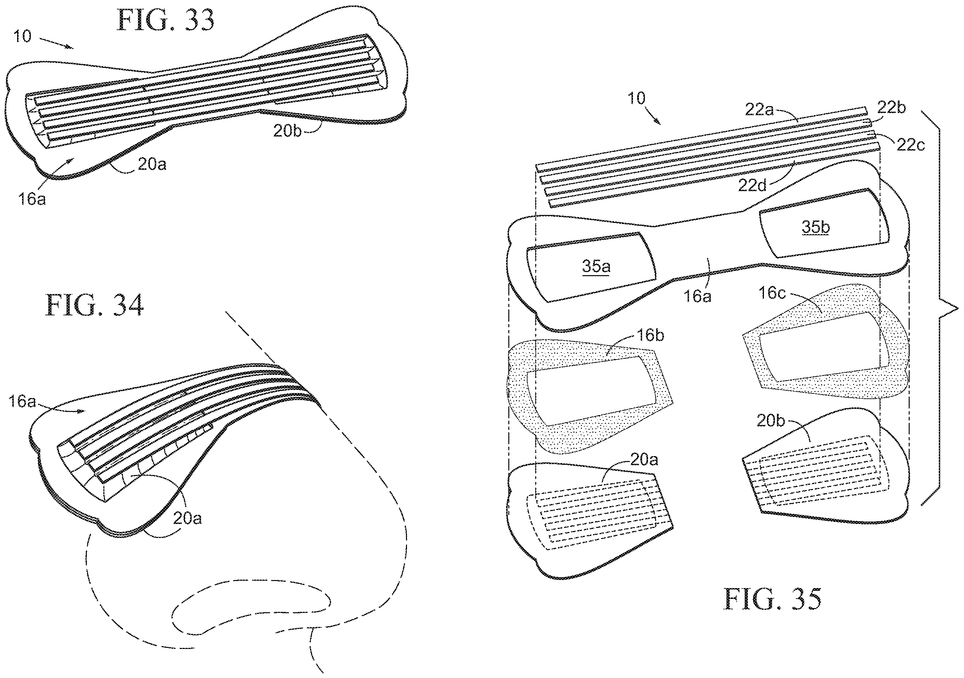

Alternatively, any number of adjacent, generally parallel resilient members may be used, up to the limitations of the properties of the preferred material (which can only be slit so narrow), or otherwise that may fit within a workable width of dilator **10.** Accordingly, it is believed that from two to about six resilient members is preferable, as particularly seen, for example, in Figs. 33-38.

Multiple resilient members typically increase the axial, torsional, flexibility of dilator **10,** which can lead to greater conformity to the irregular features and skin surface of the nose, and thus contribute to greater comfort of the dilator thereon. Torsional flexibility is enhanced by elastic membrane **20,** particularly where substantially parallel resilient members are spaced laterally apart, as discussed previously with regard to Figs. 8 and 10. That is, portions of elastic membrane **20** extending between their respective long edges may further enhance the ability of the resilient members to flex or otherwise move independently relative to each other.

Figs. 20-32 illustrate embodiments of dilator **10** in accordance with the present invention wherein the resilient layer includes multiple resilient members, at least one of which having a divergent portion extending from intermediate region **36** into at least one end region of dilator **10.** The resilient members are spaced relatively close together, side by side along substantially rectangular mid-sections, which allows intermediate region **36** to be narrower in width. Less width thereat generally corresponds to less skin-contacting surface area, which in turn may contribute to correspondingly greater user comfort.

As seen in the respective embodiments of Figs. 20-23 and 25-28, two parallel resilient members are positioned relatively close together side by side, each member having substantially rectangular mid-sections. In one embodiment, both resilient members have divergent end portions. In the other embodiment, one resilient member is substantially rectangular from end to end, and the adjacent resilient member has divergent end portions. In either case, at least one resilient member end portion diverges laterally outward, spreading spring biasing forces across the enlarged end regions of dilator **10** and thus across a greater width than if the resilient member(s) remained substantially rectangular from end to end.

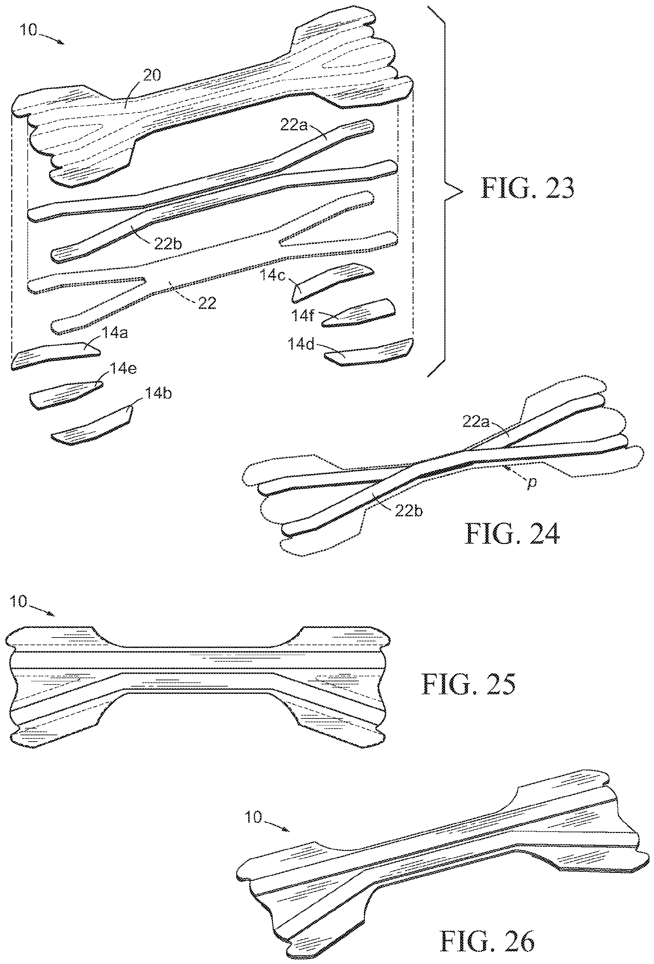

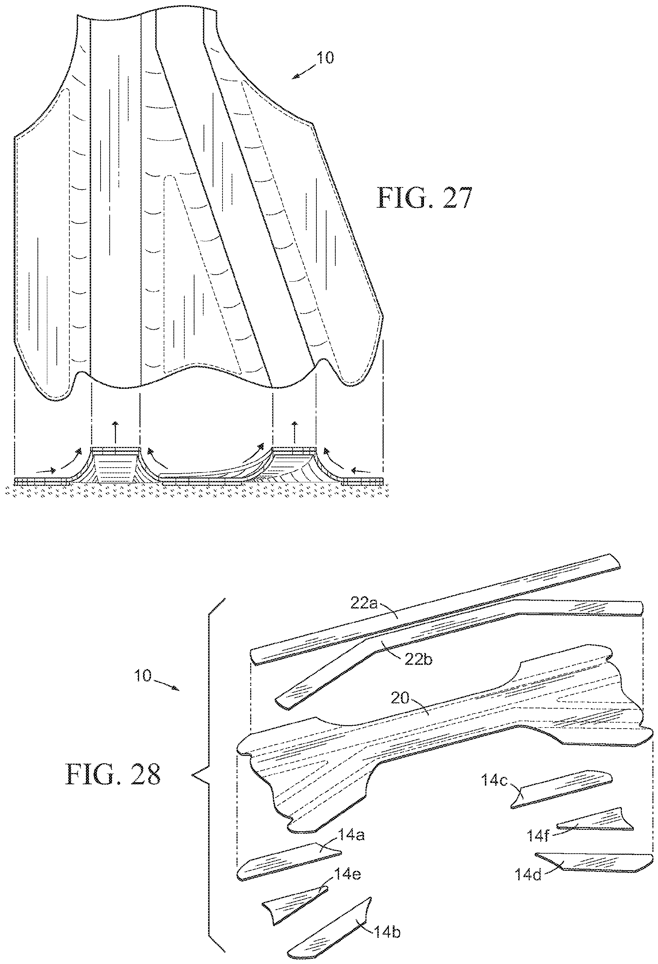

Enlarged end regions **32** and **34** each include three discrete engagement contact points, in the form of individual contact members **14a-14f,** as particularly seen in Figs. 23 and 28. (By comparison, Fig. 3, for example, illustrates two engagement contact points at each end region.) Contact members **14** are spaced apart across the width of each end region, positioned outboard the resilient members' long edges. Figs. 22 and 27 include broken lines that indicate alignment of the resilient member terminal ends to the spaces between contact members **14.**

The cross-sectional and end edge views, respectively, of Figs. 22 and 27 further illustrate resilient member terminal ends pulling upward at the two discrete areas located between, and defined by, the three spaced apart contact members **14.** Directional arrows indicate vertical separation of the resilient member terminal ends from skin surface **6** together with some shear forces extending roughly parallel thereto, as discussed hereinbefore.

Fig. 23 illustrates by dashed lines that resilient members **22a** and **22b** may be formed as a single unit, resilient member **22,** having divergent spring finger components extending outward from a common center. Alternatively, Fig. 24 illustrates that resilient members **22a** and **22b** may overlap, one onto another, which may allow the dilator periphery ***p*** to be narrower still at the intermediate region thereof. It will be apparent to the skilled artisan that the three resilient member configurations-side by side, overlapping or spring finger-may be generally similar in overall peripheral shape, and thus may be incorporated into similar peripheral configurations of dilator **10.**

Resilient member terminal ends may be set inboard from the dilator end edges, as seen, for example, in Figs. 12-16 and 29-37. Contact member **14** may thus extend around the peripheral edge of each dilator end region, as discussed hereinbefore. Divergent resilient member end portions position adjacent resilient member terminal ends laterally farther apart than would be the case with parallel rectangular resilient members.

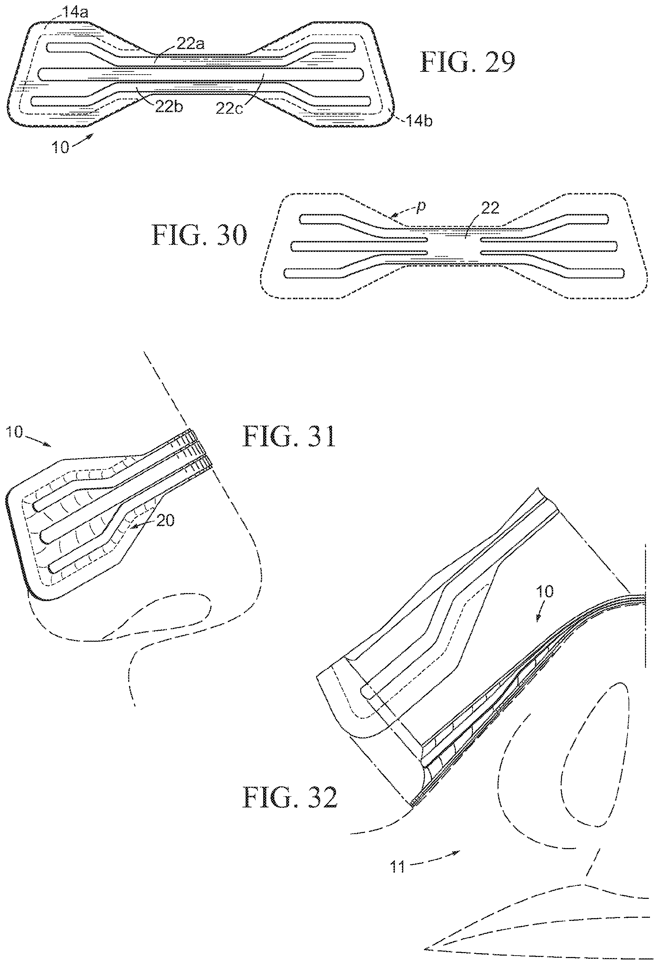

A middle resilient member may be configured to have a greater spring biasing force, for example, than the resilient members positioned adjacent to each side thereof. Fig. 32 particularly illustrates middle resilient member end portions and terminal ends thereof separating farther from the surface plane of the nose than the outer resilient members adjacent to each side. Broken lines extend between the long edge view and a fragmentary plan view to more particularly illustrate alignment of the resilient member terminal ends. An additional broken line indicates the centerline of nose **11.**

Figs. 33-38 illustrate additional embodiments of dilator **10** in accordance with the present invention. As seen in Figs. 33-35, terminal ends and end portions of a plurality of resilient members are secured to elastic membranes **20a** and **20b** through openings, **35a** and **35b,** at each end region, respectively, of dilator **10.** Fig. 34 particularly shows resilient member end portions pulling upward and tensioning that portion of elastic membrane **20a** extending through opening **35a**. (Accordingly, reference character **20a** in Fig. 34 points to two portions of the same elastic membrane: that which extends through opening **35,** and that which is secured to the underside of bonding member **16a.)** In this manner, a portion of elastic membrane **20** thus comes into contact with the skin surface.

Fig. 35 more particularly illustrates openings **35a** and **35b** formed in bonding member **16a.** Elastic membranes **20a** and **20b** are secured to the underside (the skin-facing side) of bonding member **16a** via bonding members **16b** and **16c**. Bonding members **16b** and **16c** define a surface area extending around the periphery of openings 35. Elastic membrane **20** is also secured to the end portions of resilient members **22a-22d** positioned over openings **35** on the opposite, outer, side of bonding member **16a**.

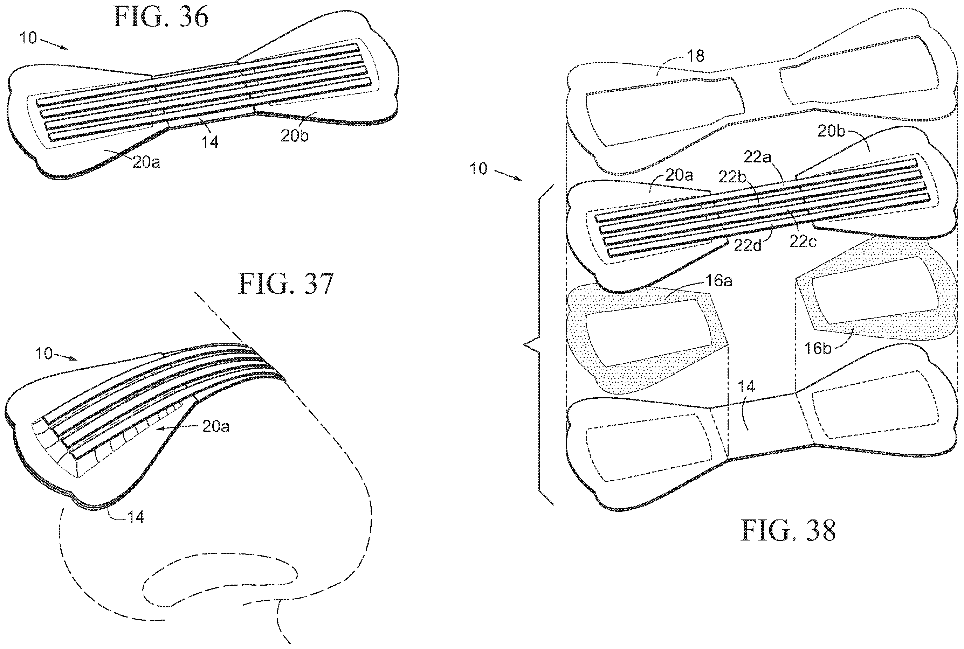

Fig. 35 particularly shows bonding member **16a** as a single piece that substantially defines the periphery of dilator **10.** (Bonding member **16a** could alternatively be bisected into two parts, similar to the peripheral shape, for example, of elastic membranes **20a** and **20b.)** Similarly, elastic membranes **20a** and **20b** could be combined into a single unit. And as seen, for example, in Fig. 38, contact member **14** may be in the form of a base layer, as described hereinbefore.

Figs. 36-38 illustrate an alternative configuration to that of Figs. 33-35 wherein elastic membranes **20a** and **20b** may be affixed to the outer, or top, side of contact member **14,** secured thereto around a perimeter defined by bonding members **16a** and **16b.** Elastic membranes **20a** and **20b** (which may be combined into a single unit) are thus interposed between contact member **14** and resilient members **22,** as particularly seen in Fig. 38. Fig. 38 further illustrates optional cover member **18,** depicted in dashed lines, extending around the peripheral long edges of dilator **10** and across the width thereof at intermediate region **36,** similar to the arrangement discussed previously with regard to Figs. 2 and 3.

Openings (as shown in Fig. 35) are not required, since bonding members **16a** and **16b** secure elastic membranes **20a** and **20b** to portions of contact member **14** extending around a perimeter. Said perimeter leaves an unsecured area capable of separating from the surface of contact member **14.** Fig. 37 particularly illustrates resilient member end portions pulling upward, tensioning the unsecured portion of elastic membrane **20a,** to the extent the elasticity of elastic membrane **20** permits, as it separates from the surface plane of contact member **14.** Dashed lines in Fig. 37 represent the margin between where elastic membrane **20a** is secured to contact member **14,** and the unsecured portion capable of separating therefrom.

The foregoing descriptions and illustrations are intended to reveal the scope of the present invention and should not be interpreted as limiting, but rather as illustrative of the inventive concepts and techniques thereof. Those skilled in the art to which the present invention is directed will appreciate that changes, modifications and alterations may be made-each such change, modification and alteration intended to be fully covered hereby-without departing from the scope of the invention, as defined by the appended claims.

## Claims

1. A nasal dilator, comprising:
an engagement element for engaging the dilator (10) to a skin surface (6) of a nose (11) of a user;
a resilient element comprising at least one resilient member (22) in at least one resilient layer, the at least one resilient member (22) having substantial in-plane rigidity and out-of-plane flexibility so as to provide resilient spring biasing forces when the dilator is in use on the nose (11), **characterized in that**
the resilient element further comprises an elastic membrane (20) having in-plane elasticity for providing stretching and tensioning forces generally parallel to its surface plane;
the elastic membrane (20) connects the engagement element to the resilient element such that the stretching and tensioning forces extend between the engagement element and the at least one resilient member (22) when the dilator is in use on the nose (11), and
said elastic membrane allows terminal ends of said resilient member to lift away from the surface of the nose.

2. The nasal dilator (10) as in claim 1, wherein a first portion of the elastic membrane (20) forms at least a portion of the engagement element, and a second portion of the elastic membrane (20) interconnects the engagement element and the at least one resilient member (22).

3. The nasal dilator (10) as in claim 1, wherein a portion of a surface of the elastic membrane (20) engages the skin surface (6) when the dilator is in use on the nose (11).

4. The nasal dilator (10) as in claim 1, wherein the elastic membrane is substantially coextensive with a periphery (p) of the dilator (10).

5. The nasal dilator (10) as in claim 1, further comprising at least one bonding layer comprising at least one bonding member (16).

6. The nasal dilator (10) as in claim 1, wherein the engagement element comprises at least one contact member (14) in at least one contact layer for engaging the dilator to the nose (11).

7. The nasal dilator (10) as in claim 6 wherein the at least one contact member (14) and the at least one resilient member (22) are engaged to a same surface of the elastic membrane (20); or
the at least one contact member (14) is engaged to a first surface of the elastic membrane, and the at least one resilient member (22) is engaged to an opposite surface of the elastic membrane.

8. The nasal dilator (10) as in claim 6 wherein a first surface of at least one contact member (20) is engaged to the skin surface (6) of the nose (11), and an opposite surface is engaged to the elastic membrane (20).

9. The nasal dilator (10) as in claim 6 wherein the at least one contact layer (14) is selected from the group consisting of:
a) a plurality of contact members (14) positioned at discrete engagement contact points **(*e*)** in at least one end region (32, 34) of the dilator (10);
b) at least one contact member (14) corresponds, at least in part, to a periphery of an end region (32, 34) of the dilator (10);
c) the at least one contact member (14) defines a periphery (p) of the dilator (10);
d) the at least one contact member (14) is formed as a base layer; or
e) a contact member (14) is formed as a base layer that is coextensive with a periphery (***p***) of the dilator (10).

10. The nasal dilator (10) as in claim 1, wherein the at least one resilient member (22) is selected from the group consisting of:
a) at least one substantially oblong resilient member (22);
b) a plurality of substantially parallel resilient members (22);
c) a plurality of spring fingers extending from a common center of at least one resilient member (22);
d) a resilient member (22) having at least one divergent end portion extending away from another portion that extends substantially parallel to a long axis of the dilator (10); or
e) two resilient members (22) that overlap at least partially onto one another.

11. The nasal dilator (10) as in claim 1 wherein the resilient element comprises between two and six substantially parallel resilient members (22) spaced laterally apart; and
a portion of the elastic membrane (20) extends between long edges at least two adjacent resilient members (22) so as to enhance torsional flexibility and the ability of the resilient members (22) to flex or otherwise move independently relative to each other.

12. The nasal dilator (10) as in claim 1 wherein at least one terminal end of the at least one resilient member (22) separates from the skin surface (6) when the dilator is engaged thereon, the separating and the stretching and tensioning forces together transforming at least a portion of the spring biasing forces from peel forces to shear forces; and
the separating further creating reduced contact area between an underside of the dilator (10) and skin surface (6) engaged by the engagement element, the reduced contact area being within a range of from about 25% to about 70% of a total plan-view area of the dilator (10).

13. The nasal dilator (10) as in claim 1, wherein when the dilator (10) is engaged to the skin surface (6):
a) greater peel forces are exerted by the at least one resilient member (22) than by the elastic membrane (20);
b) greater shear forces are exerted by the elastic membrane (20) than by the at least one resilient member (22); and
c) at least a portion of the stretching and tensioning forces extend at least roughly parallel to the skin surface (6).

14. The nasal dilator as in claim 1, further comprising a cover member (18).

## Patentansprüche

1. Nasaler Dilatator, umfassend:
ein Eingriffselement, um den Dilatator (10) mit einer Hautoberfläche (6) einer Nase (11) eines Benutzers in Eingriff zu bringen;
ein elastisches Element, das mindestens ein elastisches Element (22) in mindestens einer elastischen Schicht umfasst, wobei das mindestens eine elastische Element (22) eine wesentliche Steifigkeit in der Ebene und eine Flexibilität außerhalb der Ebene aufweist, um elastische Federvorspannkräfte bereitzustellen, wenn der Dilatator auf der Nase (11) verwendet wird, **dadurch gekennzeichnet, dass**
das elastische Element ferner eine elastische Membran (20) mit Elastizität in der Ebene umfasst, um Dehnungs- und Spannungskräfte im Allgemeinen parallel zu ihrer Flächenebene bereitzustellen,
die elastische Membran (20) das Eingriffselement mit dem elastischen Element verbindet, sodass sich die Dehnungs- und Spannungskräfte zwischen dem Eingriffselement und dem mindestens einen elastischen Element (22) erstrecken, wenn der Dilatator an der Nase (11) verwendet wird, und
die elastische Membran es den terminalen Enden des elastischen Elements ermöglicht, sich von der Oberfläche der Nase abzuheben.

2. Nasaler Dilatator (10) nach Anspruch 1, wobei ein erster Abschnitt der elastischen Membran (20) mindestens einen Abschnitt des Eingriffselements bildet und ein zweiter Abschnitt der elastischen Membran (20) das Eingriffselement und das mindestens eine elastische Element (22) miteinander verbindet.

3. Nasaler Dilatator (10) nach Anspruch 1, wobei ein Abschnitt einer Fläche der elastischen Membran (20) an der Hautoberfläche (6) eingreift, wenn der Dilatator auf der Nase (11) verwendet wird.

4. Nasendilatator (10) nach Anspruch 1, wobei die elastische Membran im Wesentlichen mit einem Umfang (*p*) des Dilatators (10) deckungsgleich ist.

5. Nasaler Dilatator (10) nach Anspruch 1, ferner umfassend mindestens eine Haftschicht, die mindestens ein Haftelement (16) umfasst.

6. Nasaler Dilatator (10) nach Anspruch 1, wobei das Eingriffselement mindestens ein Kontaktelement (14) in mindestens einer Kontaktschicht umfasst, um den Dilatator mit der Nase (11) in Eingriff zu bringen.

7. Nasaler Dilatator (10) nach Anspruch 6, wobei das mindestens eine Kontaktelement (14) und das mindestens eine elastische Element (22) mit derselben Fläche der elastischen Membran (20) in Eingriff stehen; oder
das mindestens eine Kontaktelement (14) mit einer ersten Oberfläche der elastischen Membran in Eingriff steht und das mindestens eine elastische Element (22) mit einer gegenüberliegenden Fläche der elastischen Membran in Eingriff steht.

8. Nasaler Dilatator (10) nach Anspruch 6, bei dem eine erste Fläche von mindestens einem Kontaktelement (20) mit der Hautoberfläche (6) der Nase (11) in Eingriff steht und eine gegenüberliegende Fläche mit der elastischen Membran (20) in Eingriff steht.

9. Nasaler Dilatator (10) nach Anspruch 6, wobei die mindestens eine Kontaktschicht (14) aus der Gruppe ausgewählt ist, bestehend aus:
a) einer Vielzahl von Kontaktelementen (14), die an diskreten Eingriffskontaktpunkten (e) in mindestens einem Endbereich (32, 34) des Dilatators (10) angeordnet sind;
b) mindestens einem Kontaktelement (14), das mindestens teilweise einem Umfang eines Endbereichs (32, 34) des Dilatators (10) entspricht;
c) dem mindestens einen Kontaktelement (14), das einen Umfang (p) des Dilatators (10) definiert;
d) dem mindestens einen Kontaktelement (14), das als Basisschicht ausgebildet ist; oder
e) einem Kontaktelement (14), das als Basisschicht ausgebildet ist, die mit einem Umfang (p) des Dilatators (10) deckungsgleich ist.

10. Nasaler Dilatator (10) nach Anspruch 1, wobei das mindestens eine elastische Element (22) aus der Gruppe ausgewählt ist, bestehend aus:
a) mindestens einem im Wesentlichen länglichen, elastischen Element (22);
b) einer Vielzahl von im Wesentlichen parallelen elastischen Elementen (22);
c) einer Vielzahl von Federfingern, die sich von einer gemeinsamen Mitte von mindestens einem elastischen Element (22) erstrecken;
d) einem elastischen Element (22) mit mindestens einem abweichenden Endabschnitt, der sich von einem anderen Abschnitt weg erstreckt, der sich im Wesentlichen parallel zu einer Längsachse des Dilatators (10) erstreckt; oder
e) zwei federnden Elementen (22), die sich zumindest teilweise eines über dem anderen überlappen.

11. Nasaler Dilatator (10) nach Anspruch 1, wobei das elastische Element zwischen zwei und sechs im Wesentlichen parallele elastische Elemente (22) umfasst, die seitlich voneinander beabstandet sind; und
ein Abschnitt der elastischen Membran (20) sich zwischen den langen Kanten von mindestens zwei benachbarten elastischen Elementen (22) erstreckt, um die Torsionsflexibilität und die Fähigkeit der elastischen Elemente (22), sich zu biegen oder anderweitig unabhängig voneinander zu bewegen, zu verbessern.

12. Nasaler Dilatator (10) nach Anspruch 1, wobei sich mindestens ein terminales Ende des mindestens einen elastischen Elements (22) von der Hautoberfläche (6) trennt, wenn der Dilatator darauf eingreift, wobei die Trenn- und die Dehnungs- und Spannkräfte zusammen mindestens einen Abschnitt der Federvorspannkräfte von Schälkräften in Scherkräfte umwandeln; und
wobei das Trennen ferner einen verringerten Kontaktbereich zwischen einer Unterseite des Dilatators (10) und der Hautoberfläche (6) erzeugt, an der das Eingriffselement eingreift, wobei der verringerte Kontaktbereich innerhalb eines Bereichs von etwa 25 % bis etwa 70 % eines Gesamtdraufsichtbereichs des Dilatators (10) liegt.

13. Nasaler Dilatator (10) nach Anspruch 1, wobei, wenn der Dilatator (10) mit der Hautoberfläche (6) in Eingriff steht:
a) größere Schälkräfte durch das mindestens eine elastische Element (22) als durch die elastische Membran (20) ausgeübt werden;
b) größere Scherkräfte durch die elastische Membran (20) als durch das mindestens eine elastische Element (22) ausgeübt werden; und
c) mindestens ein Abschnitt der Dehnungs- und Spannungskräfte sich mindestens annähernd parallel zur Hautoberfläche (6) erstrecken.

14. Nasaler Dilatator nach Anspruch 1, ferner umfassend ein Abdeckelement (18).

## Revendications

1. Dilatateur nasal, comprenant :
un élément d'engagement destiné à engager le dilatateur (10) sur une surface cutanée (6) du nez (11) d'un utilisateur ;
un élément élastique comprenant au moins un élément élastique (22) dans au moins une couche élastique, ledit au moins un élément élastique (22) comportant une rigidité dans le plan et une flexibilité hors plan substantielles de manière à fournir des forces de sollicitation de rappel élastiques lorsque le dilatateur est utilisé sur le nez (11),
**caractérisé en ce que**
l'élément élastique comprend en outre une membrane élastique (20) comportant une élasticité dans le plan pour fournir des forces d'allongement et de tension généralement parallèles à son plan de surface ;
la membrane élastique (20) relie l'élément d'engagement à l'élément élastique de sorte que les forces d'allongement et de tension s'étendent entre l'élément d'engagement et ledit au moins un élément élastique (22) lorsque le dilatateur est utilisé sur le nez (11), et
ladite membrane élastique permet aux extrémités terminales dudit élément élastique de se soulever de la surface du nez.

2. Dilatateur nasal (10) selon la revendication 1, une première partie de la membrane élastique (20) formant au moins une partie de l'élément d'engagement, et une seconde partie de la membrane élastique (20) reliant l'élément d'engagement et ledit au moins un élément élastique (22) entre eux.

3. Dilatateur nasal (10) selon la revendication 1, une partie d'une surface de la membrane élastique (20) s'engageant sur la surface cutanée (6) lorsque le dilatateur est utilisé sur le nez (11).

4. Dilatateur nasal (10) selon la revendication 1, ladite membrane élastique étant sensiblement coextensive avec la périphérie (p) du dilatateur (10).

5. Dilatateur nasal (10) selon la revendication 1, comprenant en outre au moins une couche de liaison comprenant au moins un élément de liaison (16).

6. Dilatateur nasal (10) selon la revendication 1, ledit élément d'engagement comprenant au moins un élément de contact (14) dans au moins une couche de contact pour engager le dilatateur avec le nez (11).

7. Dilatateur nasal (10) selon la revendication 6, ledit au moins un élément de contact (14) et ledit au moins un élément élastique (22) étant engagés sur une même surface de la membrane élastique (20) ; ou
ledit au moins un élément de contact (14) étant engagé sur une première surface de la membrane élastique, et ledit au moins un élément élastique (22) étant engagé sur une surface opposée de la membrane élastique.

8. Dilatateur nasal (10) selon la revendication 6, une première surface d'au moins un élément de contact (20) étant engagée sur la surface cutanée (6) du nez (11), et une surface opposée étant engagée sur la membrane élastique (20).

9. Dilatateur nasal (10) selon la revendication 6, ladite au moins une couche de contact (14) étant choisies dans le groupe constitué par :
a) une pluralité d'éléments de contact (14) positionnés au niveau de points de contact d'engagement discrets (e) dans au moins une zone d'extrémité (32, 34) du dilatateur (10) ;
b) au moins un élément de contact (14) correspond, au moins en partie, à la périphérie d'une zone d'extrémité (32, 34) du dilatateur (10) ;
c) ledit au moins un élément de contact (14) définit une périphérie (p) du dilatateur (10) ;
d) ledit au moins un élément de contact (14) est formé sous la forme d'une couche de base ; ou
e) un élément de contact (14) est formé sous la forme d'une couche de base qui est coextensive avec la périphérie (p) du dilatateur (10).

10. Dilatateur nasal (10) selon la revendication 1, ledit au moins un élément élastique (22) étant choisi dans le groupe constitué par :
a) au moins un élément élastique sensiblement oblong (22) ;
b) une pluralité d'éléments élastiques sensiblement parallèles (22) ;
c) une pluralité de doigts à ressort s'étendant à partir du centre commun d'au moins un élément élastique (22) ;
d) un élément élastique (22) comportant au moins une partie d'extrémité divergente s'étendant à l'opposé d'une autre partie qui s'étend sensiblement parallèlement à un axe long du dilatateur (10) ; ou
e) deux éléments élastiques (22) qui se chevauchent au moins partiellement l'un sur l'autre.

11. Dilatateur nasal (10) selon la revendication 1, ledit élément élastique comprenant entre deux et six éléments élastiques sensiblement parallèles (22) espacés latéralement ; et
une partie de la membrane élastique (20) s'étendant entre les bords longs d'au moins deux éléments élastiques adjacents (22) de manière à améliorer la flexibilité en torsion et la capacité des éléments élastiques (22) à fléchir ou sinon à se déplacer indépendamment l'un par rapport à l'autre.

12. Dilatateur nasal (10) selon la revendication 1, ladite au moins une extrémité terminale dudit au moins un élément élastique (22) se séparant de la surface cutanée (6) lorsque le dilatateur est engagé sur celle-ci, les forces de séparation et d'allongement et de tension transformant ensemble au moins une partie des forces de sollicitation de rappel des forces de décollement en forces de cisaillement ; et
ladite séparation créant en outre une zone de contact réduite entre une face inférieure du dilatateur (10) et la surface cutanée (6) engagée par l'élément d'engagement, ladite zone de contact réduite se trouvant dans une plage d'environ 25 % à environ 70 % de la zone de vue en plan totale du dilatateur (10).

13. Dilatateur nasal (10) selon la revendication 1, lorsque le dilatateur (10) est engagé sur la surface cutanée (6) :
a) des forces de décollement plus importantes étant exercées par ledit au moins un élément élastique (22) que par la membrane élastique (20) ;
b) des forces de cisaillement plus importantes étant exercées par la membrane élastique (20) que par ledit au moins un élément élastique (22) ; et
c) au moins une partie des forces d'allongement et de tension s'étendant au moins à peu près parallèlement à la surface cutanée (6).

14. Dilatateur nasal selon la revendication 1, comprenant en outre un élément de recouvrement (18).
